(19) Europäisches Patentamt
European Patent Office
Office européen des brevets

(11) Publication number: **0 080 117**
**B1**

(12) **EUROPEAN PATENT SPECIFICATION**

(45) Date of publication of patent specification: **02.12.87**

(51) Int. Cl.[4]: **C 07 D 265/34,** A 61 K 31/535

(21) Application number: **82110411.4**

(22) Date of filing: **11.11.82**

(54) **The (R,R)-enantiomer of trans-indeno(1,2-b)-1,4-oxazines, process for its preparation and pharmaceutical formulation containing it.**

(30) Priority: **20.11.81 US 323350**

(43) Date of publication of application:
**01.06.83 Bulletin 83/22**

(45) Publication of the grant of the patent:
**02.12.87 Bulletin 87/49**

(84) Designated Contracting States:
**CH DE FR GB LI NL**

(56) References cited:
**US-A-3 642 789**

**Chemical Abstracts vol. 97, no. 15, 11 October 1982, Columbus, Ohio, USA K. IORDANOVA et al. "Synthesis of 2,3,4,4a,9,9a-hexahydroindeno2,1-br-1,4-oxazine derivatives", page 722, column 2, abstract no. 127586d**

(73) Proprietor: **MERCK & CO. INC.**
**126, East Lincoln Avenue P.O. Box 2000**
**Rahway New Jersey 07065-0900 (US)**

(72) Inventor: **Jones, James H.**
**649 Midway Lane**
**Blue Bell, PA.19422 (US)**
Inventor: **McClure, David E.**
**1502 St. Andrews Way**
**Lansdale, PA. 16701 (US)**

(74) Representative: **Blum, Rudolf Emil Ernst et al**
**c/o E. Blum & Co Patentanwälte Vorderberg 11**
**CH-8044 Zürich (CH)**

**EP 0 080 117 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

Background of the Invention

The present invention concens the (R,R)-enantiomers of *trans*-2,3,4,4a,5,9b-hexahydroindeno[1,2-b]-1,4-oxazines of structural formula I

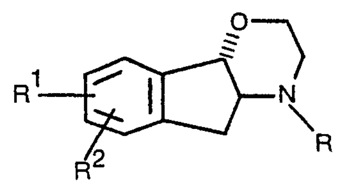

I (R,R)—enantiomer

The racemic mixture of the compounds of formula I is described by Faith et al. in US—A—3,642,789 and it is stated therein that said racemic mixture has an antidepressant activity by virtue of its reserpine antagonism and amphetamine agonism.

The (S,S)-enantiomer was isolated and it was found that said (S,S)-enantiomer has only antidepressant activity.

The optical antipode, i.e. the inventive (R,R)-enantiomer of the compound of formula I, however, quite surprisingly demonstrates the dopaminergic activity of antiparkinson agents and said (R,R)-enantiomer accordingly has a somewhat opposite effect on the central nervous system, compared with the prior art racemic mixture.

Detailed Description of the Invention

One object of the present invention is the (R,R)-enantiomers of a compound of structural formula I

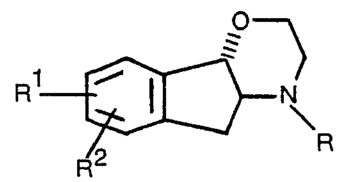

I (R,R)—enantiomer

and the pharmaceutically acceptable salts of the (R,R)-enantiomer of the compounds of formula I wherein in said formula I

R is an alkyl group having 1—4 carbon atoms, a phenyl alkyl group having 1—4 carbon atoms in the alkyl moiety, or an alkenyl group having 2—5 carbon atoms and

$R^1$ and $R^2$ are independently hydrogen, hydroxy or alkoxy groups having 1—4 carbon atoms.

The (R,R)-enantiomers of formula I and the salts thereof are new compounds.

In the compounds of formula I R is preferably methyl, ethyl, propyl, benzyl or allyl and $R^1$ and $R^2$ are preferably hydrogen, hydroxy, methoxy or ethoxy.

Specially preferred are those (R,R)-enantiomers of the compounds of formula I wherein R is methyl, ethyl or propyl and $R_1$ and $R_2$ are independently hydrogen, methoxy or hydroxy.

A further object of the present invention is a process for the preparation of the inventive (R,R)-enantiomer of the compound of structural formula I

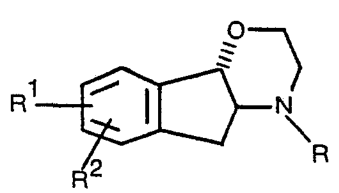

I (R,R)—enantiomer

wherein R, $R^1$ and $R^2$ are as defined or as defined above. Said process is performed by reducing the (R,R)-enantiomer of a compound of structural formula II

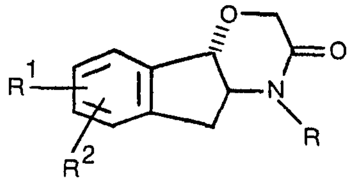

II (R,R)– enantiomer

wherein R, $R^1$ and $R^2$ are as defined in formula I.

It, however, is also possible to prepare the (R,R)-enantiomer of formula I by reducing the racemic mixture of the compounds of formula II which yields the racemic mixture of the compound of formula I from which thereafter the (R,R)-enantiomer is isolated.

A further object of the present invention is a pharmaceutical composition for the use as antiparkinson composition which comprises as pharmaceutically active antiparkinson agent a (R,R)-enantiomer of a compound of formula I according to claim 1 or a pharmaceutically acceptable salt of said compound and, furthermore, a pharmaceutical carrier.

The reduction of the (R,R)-enantiomer of the oxazinone of formula II which yields the (R,R)-enantiomer of the compounds of formula I is illustrated by the following reaction scheme:

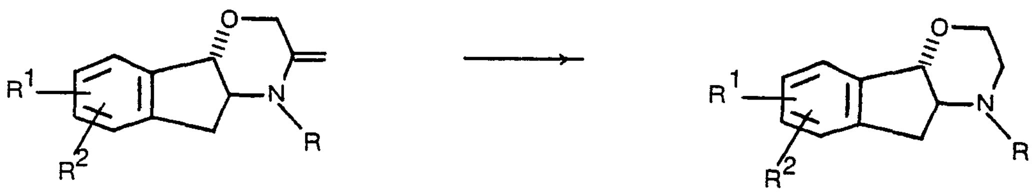

II

I

(R,R)–enantiomer

Said reduction is preferably performed with a metal hydride such as borane, in an inert organic solvent such as an ether, for example, tetrahydrofuran, 1,2-dimethoxyethane or tetrahydropyran at a temperature between about 0°C and 100°C. Operating procedures normally involve slow addition of the ketone to the reducing agent at room temperature or below, followed by heating to an elevated temperature within the stated range, preferably, the reflux temperature of the solvent, for about ½ to about 4 hours usually about 1—2 hours.

The pharmaceutically acceptable salts of the inventive (R,R)-enantiomers of formula I are preferably acid addition salts formed from a novel compound and an organic or inorganic acid recognized by the art as providing a pharmaceutically acceptable acid addition salt, such as hydrochloride, hydrobromide, dihydrogen phosphate, sulfate, citrate, pamoate, pyruvate, napsylate, isethionate, maleate and fumarate.

These salts are readily prepared by mixing solutions of equimolecular amounts of the free base compound and the desired acid in suitable solvents such as water, alcohols, ether or chloroform, followed by recovery of the product by collecting the precipitated salt or evaporation of the solvent.

The inventive pharmaceutical compositions which contain as pharmaceutically active ingredient the (R,R)-enantiomer of the compounds of formula I or a pharmaceutically acceptable salt thereof may be in any art recognized form suitable for oral use, such as tablets, troches, lozenges, aqueous or oil suspensions, dispersible powders, or granules, emulsions, hard or soft capsules, syrups, or elixirs. For intravenous and intramuscular and subcutaneous use the pharmaceutical compositions may be in any art recognized form of a sterile injectable preparation such as a sterile aqueous or oleaginous solution or suspension. The amount of active ingredient incorporated in a unit dosage of the above described pharmaceutical compositions may be from 1 to 400 mg, and preferably from 5 to 250 mg.

In order to treat Parkinsonism with an inventive pharmaceutical composition containing the new (R,R)-enantiomer of the compounds of formula I or the pharmaceutically acceptable salts thereof, the route of administration can be oral, rectal, intravenous, intramuscular, or subcutaneous. Doses of 1.0 to 200 mg/kg/day and preferably of 5.0 to 100 mg/kg day of the active ingredient to demonstrate its respective utility are generally adequate, and if preferred it can be administered in divided doses given two to four times daily.

It is to be noted that the precise unit dosage form and dosage level depend upon the requirements of the individual being treated, and consequently are left to the discretion of the therapist.

Example 1

*trans*-4-methyl 2,3,4,4a,5,9b-hexahydroindeno [1,2-b]-1,4-oxiranes

*Step A:* Preparation of *trans*-2-(N-methyl-N-acetoacetylamino)-1-indanol (IV)

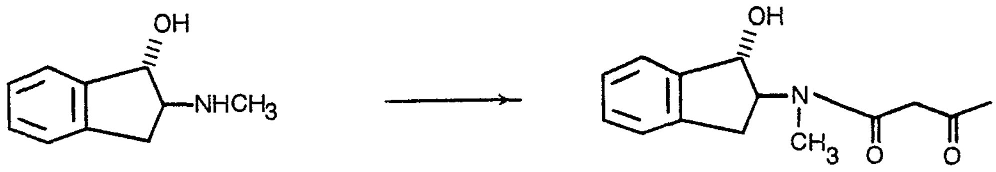

III IV

To a solution of *trans*-2-methylamino-1-indanol (4.9 g, 0.03 m) in 100 ml of ethanol was added 2.8 g (0.033 m) of diketene. After stirring about 1 hour the mixture was concentrated to dryness *in vacuo*. The residue was dissolved in acetonitrile and the solvent was evaporated *in vacuo* to 6.3 g (85%) of an oily residue which was used directly in the next step.

*Step B:* Preparation of *trans*-2-(N-methyl-N-diazoacetoacetylamino)-1-indanol (V)

IV 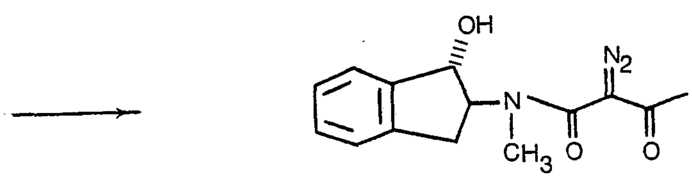

V

A mixture of the oily residue of compound IV from Step A, 100 ml of acetonitrile and 9.0 g (0.031 m) of *p*-carboxybenzenesulfonyl azide was cooled in ice and treated all at once with 9.0 ml (9.15 g, 0.06 m) of 1,8-diazabicyclo [5.4.0] undec-7-ene (DBU) with stirring. A homogeneous solution formed quickly followed by precipitation. The ice-bath was removed and stirring was continued for about 4 hours. Water was added and the mixture was extracted with methylene chloride (3×). The methylene chloride extract was back washed with water (2×), dried over anhydrous sodium sulfate and concentrated to dryness *in vacuo* leaving a residue of 7.0 g of compound V which was used directly in the next step without purification.

*Step C:* Preparation of 2-(N-diazoacetyl-N-methyl)amino-1-indanol (VI)

V 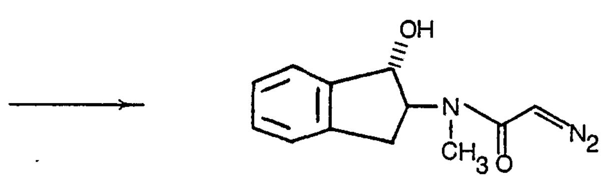

VI

The residue of structure V from Step B in 100 ml of acetonitrile was treated with 50 ml of 5% (w/v) aqueous sodium hydroxide and the mixture was stirred 5 hours at room temperature. The mixture was partitioned between added water and methylene chloride and the phases were separated. The aqueous phase was extracted again with methylene chloride and the combined methylene chloride layers were dried over anhydrous sodium sulfate. Concentration to dryness gave 4.55 g of racemic compound VI, m.p. 142—145°C.

*Step D:* Preparation of *trans*-4-methyl-4,4a,5,9b-tetrahydroindeno[1,2-b]-1,4-oxazin-3-(2H)-one (II)

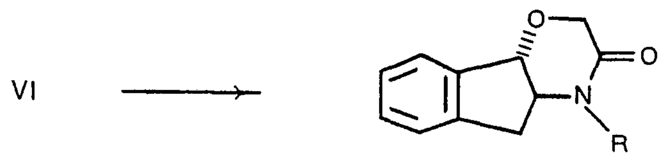

II

To a solution of 4.45 g (0.019 m) of 2-(N-diazoacetyl-N-methyl)-amino-1-indanol in 200 ml of methylene chloride cooled in an ice-bath there was added dropwise 3.4 g (2.9 ml, 0.023 m) of boron trifluoride etherate. Gas evolution began immediately and the mixture was stirred for 30 minutes. Hydrochloric acid (100 ml of 1 *N*) was added and stirring was continued for another 30 minutes. The layers were separated and the methylene chloride layer was washed with saturated aqueous sodium bicarbonate solution and water, dried over anhydrous sodium sulfate and concentrated to dryness to give 3.3 g (84%) of racemic compound II, m.p. 154—162°C.

*Step E:* Preparation of *trans*-4-methyl-2,3,4,4a,5,9b-hexahydroindeno[1,2-b]-1,4-oxazine (I)

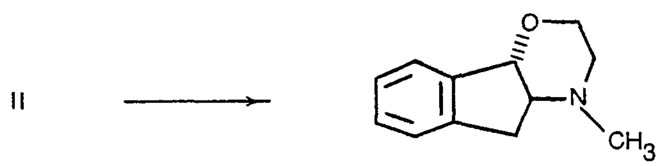

I

To a solution of 3.3 g (0.016 m) of II in 50 ml of tetrahydrofuran (THF) was added 32 ml (0.032 m) of borane (as 1 *M* $BH_3$/THF) with ice cooling. The mixture was refluxed 1 hour, cooled, treated with 50 ml of 6 *N* hydrochloric acid and heated on a steam bath until the THF had evaporated. After cooling the mixture was washed with ether, made basic with 40% (w/v) aqueous sodium hydroxide and extracted with methylene chloride. The extract was dried and concentrated to dryness *in vacuo* to give 2.4 g (42% overall for 5 steps) of racemic Compound I. Conversion to the hydrochloride gave *trans*-racemic I.HCl.$\frac{1}{4}H_2O$: m.p. 252—255°C.

Employing the procedures substantially as described in Example 1, Steps A through E, but substituting for the starting material used in Step A thereof, an equimolar amount of the (R,R)-enantiomer of *trans*-2-methylamino-1-indanol, there was prepared in comparable yield: *trans*-(R,R)-I.HCl: m.p. 260—268°C., $[\alpha]_D^{25}$ −20.38 (C, 0.52 MeOH).

Employing the procedure substantially as described in Example 1, Steps A through E, but substituting for the *trans*-2-methylamino-1-indanol used as starting material in Step A an equimolecular amount of the substituted-*trans*-2-amino-1-indanols described in Table I there are produced corresponding amounts of the substituted *trans*-2,3,4,4a,5,9b-hexahydroindeno[1,2-b]-1,4-oxazines also described in Table I, in accordance with the following reaction scheme:

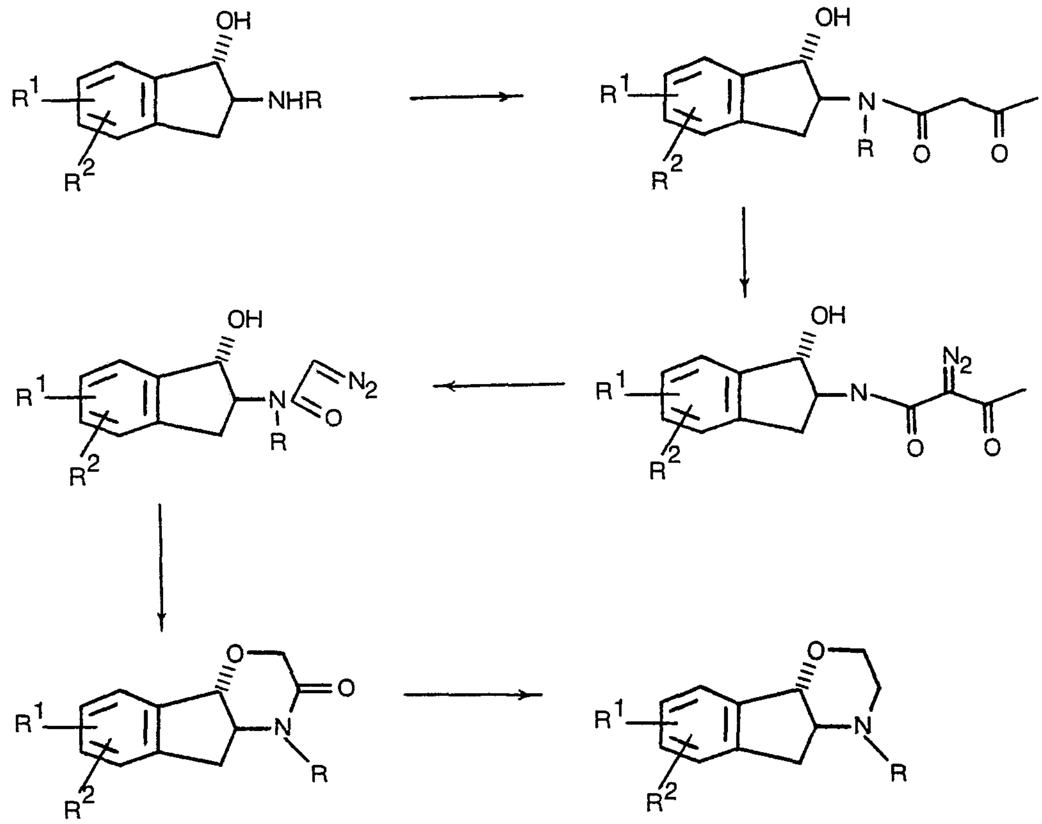

TABLE I

| Enantiomer | R | $R^1$ | $R^2$ | |
|---|---|---|---|---|
| racemic | $—C_2H_5$ | $8\text{-}OCH_3$ | H | (m.p. 243—245°C, as HCl salt) |
| (R,R) | $—C_2H_5$ | $8\text{-}OCH_3$ | H | |
| (R,R) | $—n\text{-}C_4H_9$ | H | H | |
| (R,R) | $—CH_2C_6H_5$ | H | H | |
| (R,R) | $—C_2H_5$ | $8\text{-}OCH_3$ | $7\text{-}OCH_3$ | |
| (R,R) | $i\text{-}C_3H_7$ | $7\text{-}OC_2H_5$ | $6\text{-}OC_2H_5$ | |
| (R,R) | $—CH_2CH{=}CH_2$ | H | H | |

Example 2

*trans*-4-ethyl-6-hydroxy-2,3,4,4a,5,9b-hexahydroindeno[1,2-b]-1,4-oxazine hydrobromide

*Step A:* Preparation of 4-(phenylmethoxy)-1-indanone (VII)

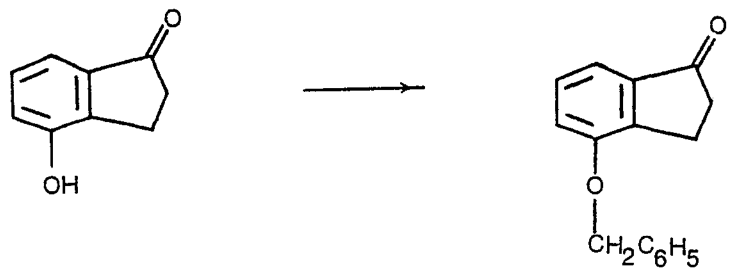

VII

To a solution of 4.44 g (0.03 m) of 4-hydroxy-1-indanone in 50 ml of dimethylformamide is added 4.15 g (0.03 m) of potassium carbonate with stirring followed by the dropwise addition of 3.8 g (0.03 m) of benzyl chloride. The reaction mixture is heated at steam bath temperature for 3 hours, then cooled and diluted with water. The aqueous mixture is extracted with ethyl acetate and the extract is washed with water, dried over anhydrous sodium sulfate and concentrated to dryness *in vacuo*. The residue is triturated with ether and concentrated to dryness to yield: 5.86 g (82%) of a brown solid, m.p. 70—73°C, sufficiently pure to be used in the next step.

*Step B:* Preparation of 2-(hydroxyimino)-4-(phenylmethoxy)-1-indanone

VII

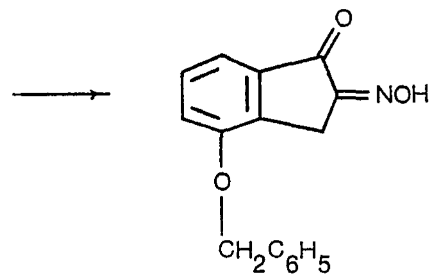

VIII

To an ice-cooled solution of 1.91 g (0.008 m) of 4-(phenylmethoxy)-1-indanone in 75 ml of anhydrous ether is added dropwise with stirring 1.2 ml (0.0088 m) of isoamyl nitrite while hydrogen chloride is simultaneously bubbled in. After stirring at room temperature for twenty minutes, the reaction mixture is partially concentrated and the solid which separates is filtered to yield 1.83 g (86%) of material sufficiently pure to be used in the next step.

*Step C:* Preparation of 2-(acetylamino)-4-hydroxy-1-indanone

VIII

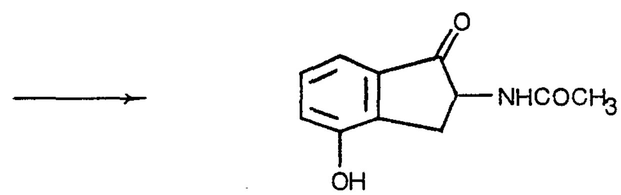

IX

A suspension of 4.94 g (0.018 m) of 2-(hydroxyimino)-4-(phenylmethoxy)-1-indanone in 35 ml each of glacial acetic acid and acetic anhydride containing 0.7 g of 10% palladium on carbon catalyst is hydrogenated at room temperature until two equivalents of hydrogen are used. The catalyst is filtered off, washed with methanol, and the combined acetic acid-alcohol filtrate is concentrated to dryness *in vacuo*. The residue is triturated with ethanol and the solid which is filtered is a roughly equal mixture of the 4-hydroxy and 4-(phenylmethoxy) compounds. The mixture is taken up in aqueous sodium hydroxide and the 4-(phenylmethoxy) compound is extracted with ethyl acetate. The basic solution is acidified with aqueous hydrochloric acid and extracted with ethyl acetate. The extract is dried over anhydrous sodium sulfate and concentrated to dryness *in vacuo* to yield the 4-hydroxy compound.

*Step D:* Preparation of 2-acetylamino-4-(methylthiomethoxy)-1-indanone (X)

IX

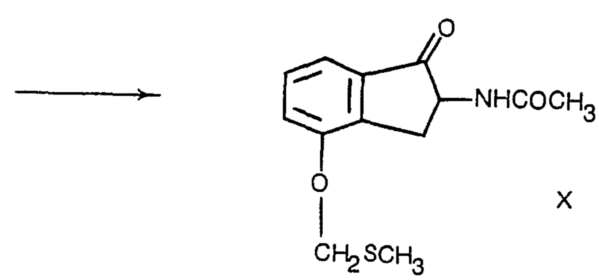

X

To 2-acetylamino-4-hydroxy-1-indanone 10.25 g (0.05 m) in 50 ml of dry HMPA under $N_2$ is added 2.6 g (0.055 m) of a 50% oil dispersion of sodium hydride in portions. After stirring at room temperature for $\frac{1}{2}$ hr, 5.3 g (0.055 m) of chloromethyl methyl sulfide is added, and the mixture is stirred overnight. Water is

added and the mixture is extracted with ethyl acetate which is back-washed with water. After drying ($Na_2SO_4$) and concentration, the desired product is obtained.

*Step E:* Preparation of *trans*-2-ethylamino-4-(methylthiomethoxy)-1-indanol (XI)

X
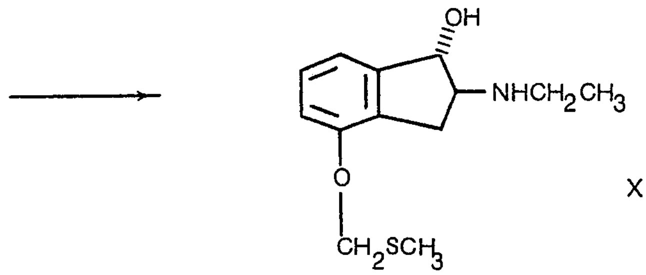
XI

The residue from Step D is dissolved in 50 ml of THF and added to 3.8 g (0.1 m) of $LiAlH_4$ in 50 ml of THF dropwise. The mixture is refluxed for one hour after completion of the addition. The mixture is cooled and sufficient saturated aqueous $Na_2SO_4$ solution is added to quench the excess $LiAlH_4$. Methylene chloride and solid anhydrous $Na_2SO_4$ is added and the mixture is filtered. Concentration provides the crude product.

*Step F:* Preparation of *trans*-2-(N-ethyl-N-acetoacetylamino)-4-(methylthiomethoxy)-1-indanol (XII)

XI
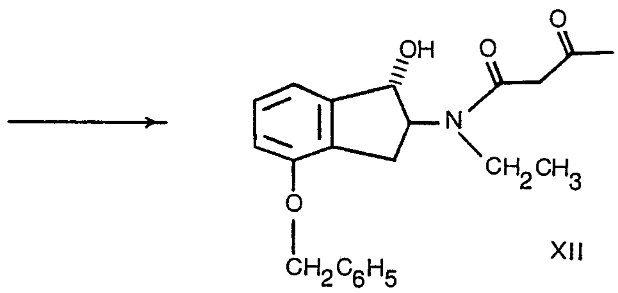
XII

Using the product from Step E and following the procedure of Example 1, Step A, with 4.6 g (0.055 m) of diketone in 200 ml ethanol, the desired product is obtained.

*Step G:* Preparation of *trans*-2-(N-ethyl-N-diazoacetoacetylamino)-4-(methylthiomethoxy)-1-indanol (XIII)

XII
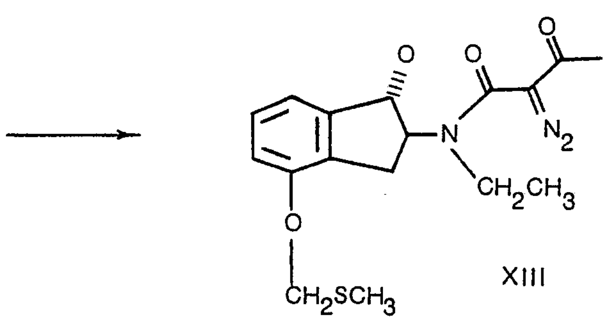
XIII

Using the product in Example 1, Step B, with 11.3 g (0.05 m) of p-carboxybenzenesulfonylazide in 150 ml of acetonitrile, the desired product is obtained.

*Step H:* Preparation of *trans*-2-(N-ethyl-N-diazoacetylamino)-4-(methylthiomethoxy)-1-indanol (XIV)

XIII
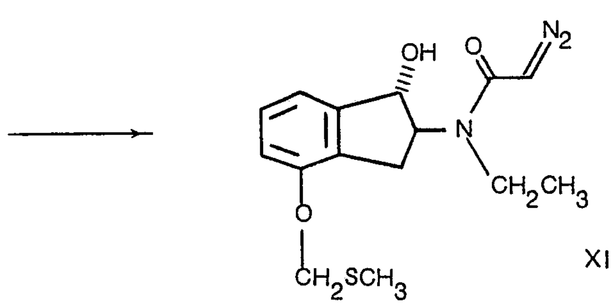
XIV

Using the product from Step G and following the procedure in Example 1, Step C, with 100 ml of acetonitrile and 50 ml of 5% (w/v) aqueous sodium hydroxide and stirring overnight, the desired product is obtained.

*Step I:* Preparation of *trans*-4-ethyl-6-(methylthiomethoxy)-4,4a,5,9b-tetrahydroindeno[1,2-b]-1,4-oxazin-3-(2H)-one (XV)

XIV

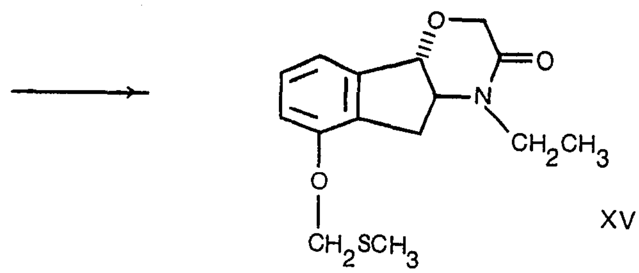

XV

Using the product from Step H and following the procedure in Example 1, Step D, with 6.4 g (5.5 ml, 0.045 m) of boron trifluoride etherate in 200 ml of methylene chloride, the desired product is obtained.

*Step J:* Preparation of *trans*-4-ethyl-6-hydroxy-4,4a,5,9b-tetrahydroindeno[1,2-b]-1,4-oxazin-3-(2H)-one (XVI)

XV

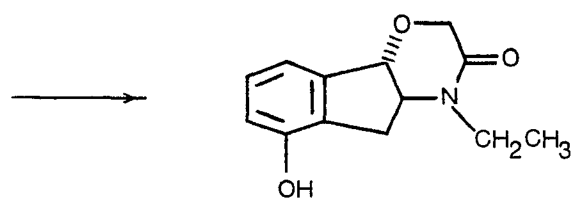

XVI

To the product from Step I in 200 ml of a 4:1 (v/v) acetonitrile-water mixture is added 12.2 g (0.045 m) of mercuric chloride. The resulting suspension is refluxed for 10 hr, diluted with methylene chloride, and filtered through celite. The organic phase is washed with water, dried over $Na_2SO_4$, and concentrated. The residue is taken up in methanol, aqueous 48% HBr is added, and the solvents are removed *in vacuo*. Methanol is added and the mixture reconcentrated several times to remove any water. Ethanol is added and ether is used to precipitate the desired HBr salt of the product.

*Step K:* Preparation of *trans*-4-ethyl-6-hydroxy-2,3,4,4a,5,9b-hexahydroindeno[1,2-b]-1,4-oxazine (I)

XVI

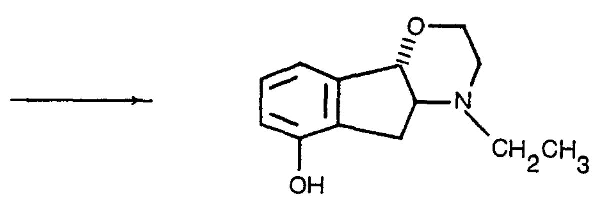

I

Using the product from Step J and following the procedure in Example 1, Step E, with 45 ml (0.045 m) of borane (as a 1*M* $BH_3$/THF solution) in 100 ml of THF, the desired product is obtained.

If compound XI, the product of Step E, is resolved into its (R,R)- and (S,S)-enantiomers and the (R,R)-enantiomer is subjected to the chemical reactions described in Steps F through K, there is produced the (R,R)-enantiomer of *trans*-4-ethyl-6-hydroxy-2,3,4,4a,5,9b-hexahydroindeno[1,2-b]-1,4-oxazine.

Employing the procedures substantially as described in Example 2, Steps A through K but substituting for the starting material used therein equimolecular amounts of the hydroxy-1-indanones described in Table II, there are produced the racemic and (R,R)-enantiomers of the substituted *trans*-2,3,4,4a,5,9b-hexahydroindeno-[1,2-b]-1,4-oxazines, also described in Table II.

TABLE II

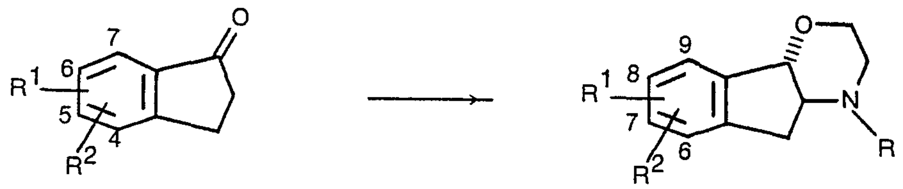

| $R^1$ | $R^2$ | R | $R^1$ | $R^2$ |
|---|---|---|---|---|
| 4-OH | | $-C_2H_5$ | 6-OH | |
| 4-OH | 5-OH | $-C_2H_5$ | 6-OH | 7-OH |
| 6-OH | | $-C_2H_5$ | 8-OH | |
| 5-OH | 6-OH | $-C_2H_5$ | 7-OH | 8-OH |
| 4-OH | | $n\text{-}C_3H_7$ | 6-OH | |
| 4-OH | | $-n\text{-}C_4H_9$ | 6-OH | |
| 7-OH | | $-C_2H_5$ | 9-OH | |

Example 3

Pharmaceutical Composition

A typical tablet containing 100 mg of active ingredient per tablet is prepared by mixing together with the active ingredient, calcium phosphate, lactose and starch in the amounts shown in the table below. After these ingredients are thoroughly mixed, the appropriate amount of magnesium stearate is added and the dry mixture blended for an additional three minutes. This mixture is then compressed into tablets.

Tablet Formula

| Ingredient | Mg. per Tablet |
|---|---|
| *Trans*-(R,R)-4-methyl-2,3,4,4a,5,9b-hexahydroindeno[1,2-b]-1,4-oxazine | 100 mg |
| Calcium phosphate | 52 mg |
| Lactose | 60 mg |
| Starch | 10 mg |
| Magnesium Stearate | 1 mg |

Similarly prepared are tablets comprising as active ingredient any of the other novel compounds described herein.

**Claims**

1. The (R,R)-enantiomer of a compound of structural formula I

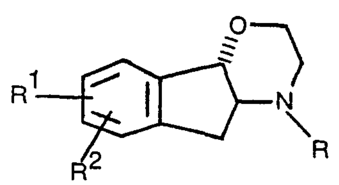

I (R,R)—enantiomer

wherein

R is an alkyl group having 1—4 carbon atoms, a phenyl alkyl group having 1—4 carbon atoms in the alkyl moiety, or an alkenyl group having 2—5 carbon atoms and

$R^1$ and $R^2$ are independently hydrogen, hydroxy or alkoxy groups having 1—4 carbon atoms or pharmaceutically acceptable salts of the R,R-enantiomer of the compounds of formula I.

2. A compound according to claim 1 characterized in that in formula I R is methyl, ethyl or propyl and $R_1$ and $R_2$ are independently hydrogen, methoxy or hydroxy.

3. A process for the preparation of the R,R-enantiomer of the compound of structural formula I

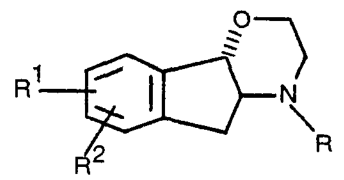

I (R,R)—enantiomer

according to claim 1, wherein R, $R^1$ and $R^2$ are as defined in claim 1, characterized in that the (R,R)-enantiomer of a compound of structural formula II

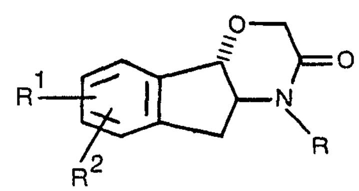

II (R,R)— enantiomer

wherein R, $R^1$ and $R^2$ are as defined in formula I is reduced.

4. Process according to claim 3 characterized in that the R,R-enantiomer according to claim 2 is prepared.

5. Process according to claim 3 or 4 characterized in that the reduction of the compound of formula II is performed with a metalhydride, preferably a borane.

6. A pharmaceutical composition for the use as antiparkinson composition characterized in that it comprises as pharmaceutically active antiparkinson agent a R,R-enantiomer of a compound of formula I according to claim 1 or a pharmaceutically acceptable salt of said compound and furthermore a pharmaceutical carrier.

7. Pharmaceutical composition according to claim 6 characterized in that it comprises as pharmaceutically active ingredient a R,R-enantiomer according to claim 2.

**Patentansprüche**

1. (R,R)-Enantiomer einer Verbindung der Strukturformel I

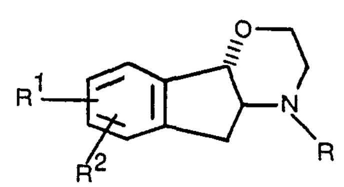

I (R,R)—Enantiomer

worin

R eine Alkylgruppe mit 1 bis 4 Kohlenstoffatomen, eine Phenylalkylgruppe mit 1 bis 4 Kohlenstoffatomen in der Alkyleinheit oder eine Alkenylgruppe mit 2 bis 5 Kohlenstoffatomen ist, und

$R^1$ und $R^2$ unabhängig Wasserstoff, Hydroxy oder Alkoxygruppen mit 1 bis 4 Kohlenstoffatomen sind oder pharmazeutisch annehmbare Salze des R,R-Enantiomeren der Verbindungen der Formel I.

2. Verbindung nach Anspruch 1, dadurch gekennzeichnet, daß in Formel I R Methyl, Ethyl oder Propyl ist, und $R_1$ und $R_2$ unabhängig Wasserstoff, Methoxy oder Hydroxy sind.

3. Verfahren zur Herstellung des R,R-Enantiomeren der Verbindung der Strukturformel I

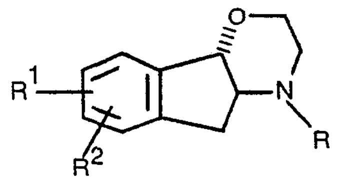

I (R,R)—Enantiomer

nach Anspruch 1, worin R, $R^1$ und $R^2$ wie in Anspruch 1 definiert sind, dadurch gekennzeichnet, daß das (R,R)-Enantiomer einer Verbindung der Strukturformel II

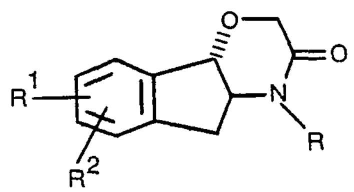

II (R,R)—Enantiomer

worin R, $R^1$ und $R^2$ wie in Formel I definiert sind, reduziert wird.

4. Verfahren nach Anspruch 3, dadurch gekennzeichnet, daß das R,R-Enantiomer nach Anspruch 2 hergestellt wird.

5. Verfahren nach Anspruch 3 oder 4, dadurch gekennzeichnet, daß die Reduktion der Verbindung der Formel II mit einem Metallhydrid, vorzugsweise einem Boran, durchgeführt wird.

6. Pharmazeutische Zusammensetzung zur Verwendung als Antiparkinson-Mittel, dadurch gekennzeichnet, daß sie als pharmazeutisch annehmbares Antiparkinson-Agens ein R,R-Enantiomer einer Verbindung der Formel I nach Anspruch 1 oder ein pharmazeutisch annehmbares Salz dieser Verbindung und weiterhin einen pharmazeutischen Träger enthält.

7. Pharmazeutische Zusammensetzung nach Anspruch 6, dadurch gekennzeichnet, daß sie als pharmazeutisch annehmbaren Bestandteile ein R,R-Enantiomer nach Anspruch 2 enthält.

**Revendications**

1. Enantiomère-(R,R) d'un composé de formule développée I

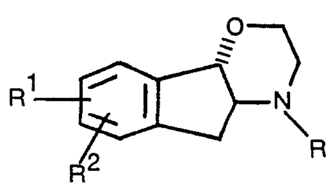

I énantiomère—(R,R)

dans laquelle R est un groupe alkyle ayant 1—4 atomes de carbone, un groupe phénylalkyle dont le fragment alkyle a 1—4 atomes de carbone ou un groupe alcényle ayant 2—5 atomes de carbone et $R^1$ et $R^2$ sont indépendamment un atome d'hydrogène, un groupe hydroxyle ou alcoxy ayant 1—4 atomes de carbone ou les sels pharmaceutiquement acceptables des énantiomères-(R,R) des composés de formule I.

2. Composé selon la revendication 1, caractérisé en ce que dans la formule I, R est le groupe méthyle, éthyle ou propyle et $R^1$ et $R^2$ sont indépendamment l'atome d'hydrogène, le groupe méthoxy ou le groupe hydroxyle.

3. Procédé pour la préparation de l'énantiomère-(R,R) du composé de formule développée I

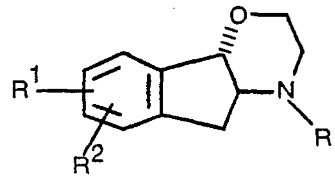

I énantiomère-(R,R)

selon la revendication 1, dans laquelle R, $R^1$ et $R^2$ sont tels que définis dans la revendication 1, caractérisé en ce que l'on réduit l'énantiomère-(R,R) d'un composé de formule développée II

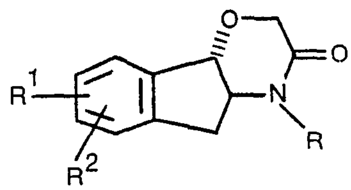

II énantiomère-(R,R)

dans laquelle R, $R^1$ et $R^2$ sont tels que définis dans la formule I.

4. Procédé selon la revendication 3, caractérisé en ce que l'on prépare l'énantiomère-(R,R) selon la revendication 2.

5. Procédé selon la revendication 3 ou 4, caractérisé en ce que l'on effectue la réduction du composé de formule II avec un hydrure métallique, de préférence un borane.

6. Composition pharmaceutique à utiliser comme composition antiparkinsonienne, caractérisée en ce qu'elle comprend, à titre d'agent antiparkinsonien pharmaceutiquement actif, un énantiomère-(R,R) d'un composé de formule I, selon la revendication 1 ou un sel pharmaceutiquement acceptable dudit composé et en outre un véhicule pharmaceutique.

7. Composition pharmaceutique selon la revendication 6, caractérisée en ce qu'elle comprend, à titre d'ingrédient pharmaceutiquement actif, un énantiomère-(R,R) selon la revendication 2.